(19) Europäisches Patentamt
European Patent Office
Office européen des brevets

(11) Veröffentlichungsnummer: **0 282 962**
**A2**

(12) **EUROPÄISCHE PATENTANMELDUNG**

(21) Anmeldenummer: **88104051.3**

(22) Anmeldetag: **15.03.88**

(51) Int. Cl.4: **B01J 19/12** , C07C 143/02 , //H01J61/18

(30) Priorität: **18.03.87 DE 3708784**

(43) Veröffentlichungstag der Anmeldung: **21.09.88 Patentblatt 88/38**

(84) Benannte Vertragsstaaten: **BE DE ES FR GB IT NL**

(71) Anmelder: **HOECHST AKTIENGESELLSCHAFT**
**Postfach 80 03 20**
**D-6230 Frankfurt am Main 80(DE)**

(72) Erfinder: **Pistorius, Rudolf, Dr.**
**Neumühlstrasse 15**
**D-6274 Hünstetten 8(DE)**
Erfinder: **Ramloch, Herbert, Dr.**
**Eichhornweg 3**
**D-6232 Bad Soden am Taunus(DE)**

(54) **Verfahren zur Erhöhung der Ausbeute bei durch Licht initiierten Reaktionen unter Verwendung von Gasentladungstrahlern.**

(57) Bei lichtinduzierten Reaktionen, insbesondere bei der Sulfoxidation von Paraffinen zur Herstellung von sek. Alkansulfonaten unter der Einwirkung von UV-Licht wurde gefunden, daß man diese Verfahren verbessern kann, wenn man die bei solchen lichtinduzierten Reaktion benutzten Gasentladungsstrahler stufenlos regelbar ausstattet.

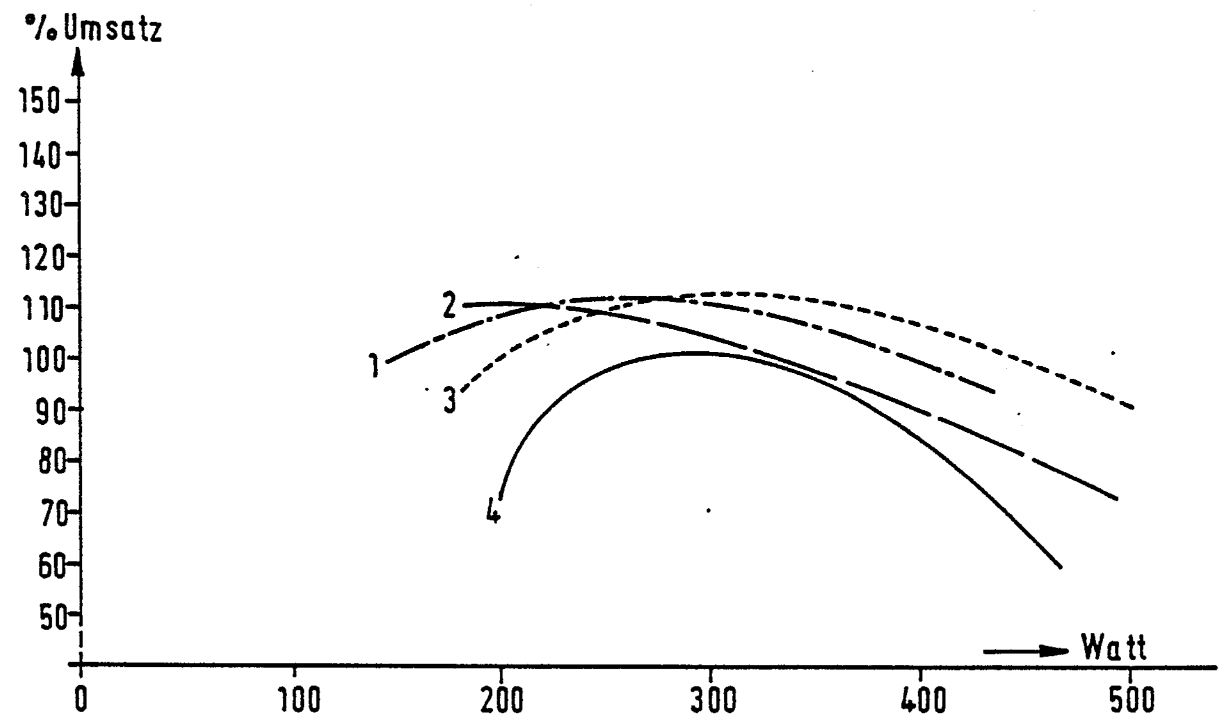


EP 0 282 962 A2

# Verfahren zur Erhöhung der Ausbeute bei durch Licht initiierten Reaktionen unter Verwendung von Gasentladungstrahlern

Bei großtechnisch durchgeführten, durch Licht katalysierten chemischen Reaktionen eignen sich vorzugsweise Quecksilber-Hcchdruckbrenner, weil mit ihnen die für die wirtschaftlich ausreichende Raumzeitausbeuten erforderlichen hohen Strahlungsdichten an UV-Licht mit dem geringsten Aufwand erzielt werden können. Die resultierende Produktionsleistung hänge dabei von vielen Parametern ab, in erster Linie jedoch von der Strahlungsemission der Lampe.

Bei der Sulfoxidation von Paraffinen können alle im UV-Bereich emittierten Wellenlängen einer Quecksilber-Hochdrucklampe die Reaktion initiieren (siehe hierzu: J. G. Calvert und Mitarb., J. Am. Chem. Soc. 93 (1971), 3115-3128). Die Menge der absorbierten Lichtquanten hängt dabei einerseits ab von der Eindringtiefe der Strahlung, die von ihrer Wellenlänge bestimmt und nach dem Lambert'schen Gesetz exponentiell geschwächt wird, andererseits vom Stoffaustausch in der bestrahlten Zone. Beim sogenannten "Licht-Wasser-Verfahren" (siehe hierzu: H. Ramloch, G. Täuber, Chemie in unserer Zeit, 13 (1979), Nr. 5, 157-162) reagieren Paraffin, Schwefeldioxid, Sauerstoff und Wasser initiiert durch UV-Licht zu Alkansulfonsäure und Schwefelsäure nach der Summengleichung:

$$RH + 2SO_2 + O_2 + H_2O \xrightarrow{h\nu} RSO_3H + H_2SO_4$$

Bei großtechnisch und insbesondere bei vollkontinuierlich durchgeführten photochemischen Prozessen wird das Reaktionsgemisch mit mehreren UV-Lampen bestrahlt. Die jeweils benötigte Strahlungsleistung kann dabei höchstens nur so eingestellt werden, wie insgesamt Lampen installiert sind, so daß die Anpassung der gewünschten Strahlungsleistung nur durch das Ein-bzw. Ausschalten einer bestimmten Anzahl der vorhandenen Lampen geregelt werden kann. Solche Ein-und Ausschaltvorschläge wirken sich aber nachteilig auf die Lebensdauer der Quecksilber-Hochdrucklampen aus.

Es wurde nun gefunden, daß man diese Schaltvorgänge vermeiden und die Ausbeute bei durch Licht initiierten Reaktionen verbessern kann, wenn man die dabei benutzten Gasentladungsstrahler stufenlos regelbar ausgestaltet. Für diese stufenlose Regelung der Gasentladungsstrahler, hier insbesondere für Quecksilber-Hochdruckbrenner, eignet sich besonders eine regelbare Gleichspannungsversorgung, wie sie in EP-A 0 179 376 beschrieben ist. Diese Gleichspannungsversorgung besteht aus einer oder mehreren ungeregelten Spannungsquellen und einer mit diesen in Reihe geschalteten veränderlichen Spannungsquelle. In einer speiziellen Ausgestaltung kann die veränderliche Spannungsquelle stufenlos durch Veränderung ihrer Amplitude oder durch Veränderung ihrer Einschaltdauer regelbar sein. Mit dieser Gleichspannungsversorgung können die Aus-und Einschaltvorgänge aller Lampen vermieden werden und genau der gewünschte Strahlungsfluß eingestellt werden.

Es wurde außerdem gefunden, daß sich eine solche regelbare Gleichspannungsversorgung besonders eignet für die Erhöhung der Ausbeute bei der Herstellung sek. Alkansulfonaten durch Sulfoxidation von n-Paraffinen unter Einwirkung von UV-Licht, das durch Quecksilber-Hochdurckbrenner erzeugt wird. Stattet man diese UV-Brenner mit einer solchen regelbaren Gleichspannungsversorgung aus, so findet man bei abnehmender elektrischer Leistungsaufnahme eine steigende Lampenstromausbeute (kg/h Produkt pro kW), bis ein Optimum erreicht wird, danach fällt die Lampenstromausbeute wieder ab. Hierbei wurde überraschend gefunden, daß bei der Sulfoxidation von Paraffinen die Lage und Höhe des Optimums der Lampenstromausbeute durch geeignete Dotierungen der Quecksilberhochdruckbrenner mit z.B. Blei, Thallium, Gallium u.a. stark beeinflußt werden kann.

Durch die stufenlos regelbare Lampenstromversorgung werden die sonst üblichen Aus-und Einschaltvorgänge zur Regelung des Strahlungsflusses im Reaktor vermieden. Dies führt durch die längere Lebensdauer der Strahler zu wirtschaftlichen Vorteilen. Überraschenderweise werden die Herstellkosten, z.B. bei der Sulfoxidation von Paraffinen, vor allem dadurch gesenkt, daß es mit der stufenlos regelbaren Schaltung gelingt, unabhängig von der gerade benötigten Anlageleistung, die Reaktion immer mit dem geringsten elektrischen Stromverbrauch durchzuführen.

Der Erfindungsgegenstand soll an den folgenden Beispielen, hier exemplarisch an der Sulfoxidation von Paraffin nach dem Licht-Wasser-Verfahren, dargestellt werden, ohne ihn weiter einzugrenzen.

Verwendet wurde in allen Fällen das gleiche $C_{14}$-$C_{17}$-n-Paraffin, um den Einfluß durch unterschiedliche Paraffinqualitäten auszuschließen.

Ebenso wurden sämtliche Umsetzungen in der gleichen Sulfoxidationsanlage unter weitestgehend identischen Bedingungen vorgenommen:

Reaktionstemperatur: 33-35°C
Gaszusammensetzung $SO_2$:$O_2$ = 2:1
Begasungsstrom: 800 l/h
UV-Tauchlampe: TQ 400 (Heraeus)
äußeres Lampenhüllrohr: ∅ außen 40 mm, Länge 300 mm
Reaktorvolumen: ca. 10 l
Abscheidervolumen: ca. 6 l
Kreisparaffinstrom: ca. 80 l/h

Die kontinuierlich zugegebene Wassermenge wurde so dosiert, daß das sich im Abscheider abtrennende Reaktionsgemisch, der sogenannte Extrakt, zu 40 % aus Wasser bestand. Variiert wurde von Versuch zu Versuch lediglich die Aufnahme von elektr. Energie der verschiedenen UV-Brenner.

Im dem nachfolgenden Bild wird die erzeugte Alkansulfonsäuremenge in Abhängigkeit von der verbrauchten Energie der jeweiligen Brenner gezeigt, wobei die mit der reinen Quecksilberhochdrucklampe (Kurve 1) bei 150 Watt erzeugte Menge von 3,8 kg Alkansulfonat-Na pro KW als 100 % gesetzt wurde. Die Kurven 2, 3 und 4 zeigen demgegenüber die entsprechenden Werte bei Verwendung von Quecksilberhochdrucklampen, die mit Blei (2), Thallium (3) bzw. Gallium (4) dotiert sind. Man sieht hier deutlich die Verschiebung des Maximums durch die Dotierung.

## Ansprüche

1. Verfahren zur Erhöhung der Ausbeute bei durch Licht initiierten Reaktionen unter Verwendung von Gasentladungsstrahlern, dadurch gekennzeichnet, daß man diese Gasentladungsstrahler stufenlos regelbar ausgestaltet.

2. Verfahren nach Anspruch 1, dadurch gekennzeichnet, daß man die stufenlose Regelung der Gasentladungsstrahler mit einer regelbaren Gleichspannungsversorgung vornimmt, die aus einer oder mehreren ungeregelten Spannungsquellen und einer mit diesen in Reihe geschalteten veränderlichen Spannungsquellen besteht.

3. Verfahren nach Anspruch 1 zur Erhöhung der Ausbeute bei der Sulfoxidation von n-Paraffinen.

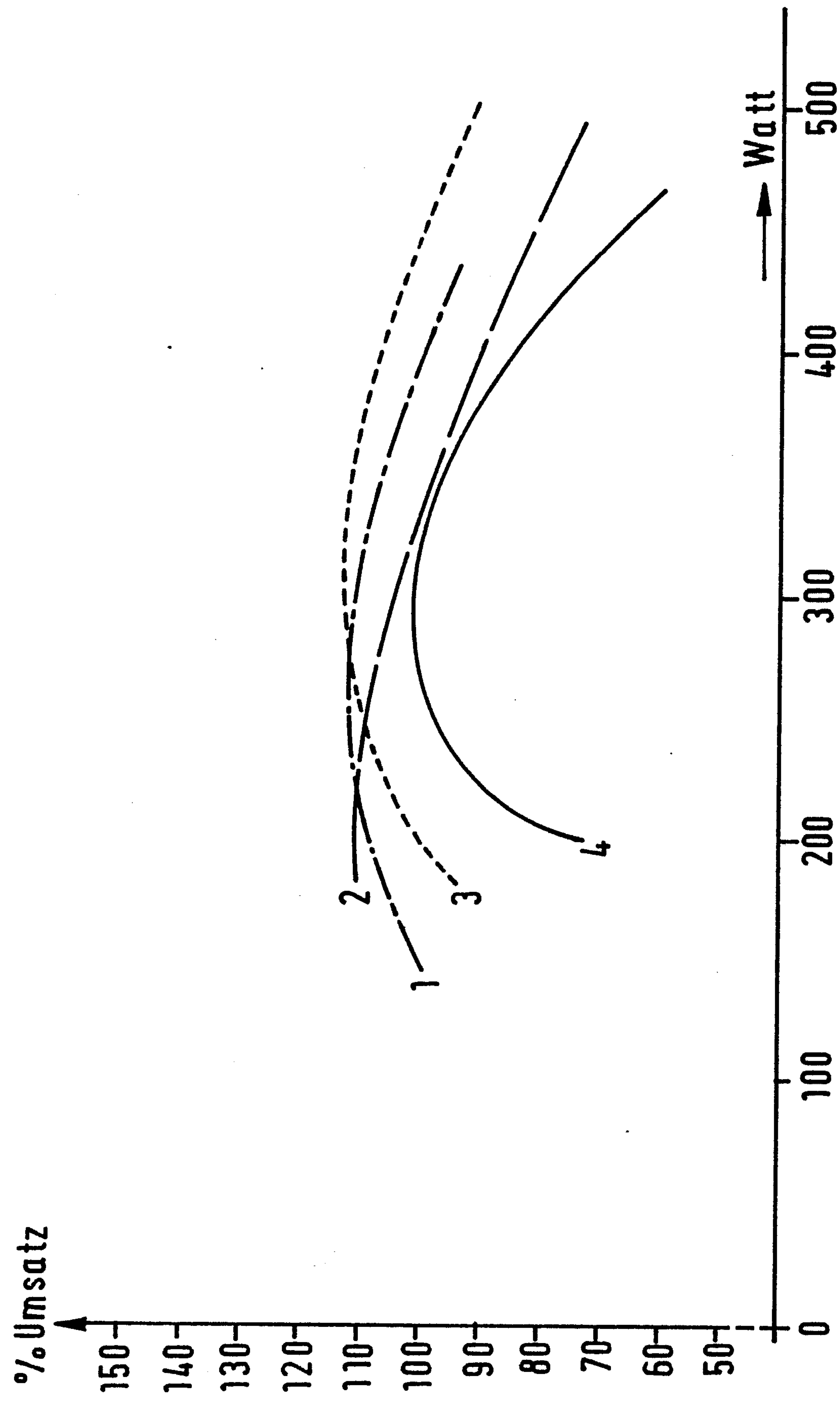
% Umsatz
150
140
130
120
110
100
90
80
70
60
50
0
100
200
300
400
500
Watt
1
2
3
4